# EUROPEAN PATENT APPLICATION

(11) **EP 4 810 520 A1**
(43) Date of publication of application: **23.09.2026**
(21) Application number: 23958513.6
(22) Date of filing: 14.11.2023
(51) Int. Cl.: C07K 16/40, C12N 15/13, A61K 39/395, A61P 35/00, C07K 16/46, C07K 16/28

(54) **ANTI-PD-1 ANTIBODY, AND BIOLOGICAL MATERIAL AND PRODUCT COMPRISING SAME**

(71) Applicant: ABlink Biotech Co., Ltd., Chengdu, Sichuan 610093 (CN)
(72) Inventor: LI, Xinlei, Chengdu, Sichuan 610093 (CN); ZENG, Xin, Chengdu, Sichuan 610093 (CN); LIU, Jianghai, Chengdu, Sichuan 610093 (CN)
(74) Representative: Cabinet Beau de Loménie
(86) International application number: PCT/CN2023/131416
(87) International publication number: WO 2025/102227

(57) **Abstract**

The present application relates to the technical field of biomedicine, and in particular to an anti-PD-1 antibody, and a biological material and product comprising same. The anti-PD-1 antibody of the present invention can effectively block the binding of PD-1 to PD-L1, and is used for preventing and/or treating cancer.

## Description

### Technical Field

The present application relates to the field of biomedicine technology, and specifically to an anti-PD-1 antibody and its biological materials and products.

### Background Art

During the occurrence and development of tumors, there are many molecular regulatory mechanisms between immune cells and tumor cells, which have a negative impact on the normal function of immune cells, thereby promoting the growth and metastasis of tumor cells. Among them, immune checkpoints play a key role. Programmed death-1 (PD-1, or CD279) and PD-1 ligand 1 (PDL1) are a pair of important checkpoint molecules that help tumor cells evade the elimination by cytotoxic T lymphocytes (CTLs). When T cells interact with cancer cells, PD-1 (expressed on the surface of T cells) binds and reacts with PD-L1 (overexpressed on various cancer cells, antigen-presenting cells, and endothelial cells). The formation of the PD-1/PD-L1 complex negatively regulates the activation and effector function of T cells and may lead to tumor immune evasion. Therefore, blocking the PD-1/PD-L1 signaling pathway can restore the cytotoxic activity of tumor antigen-specific T cells, which is widely regarded as a revolutionary therapy for a variety of malignant tumors. The FDA has approved several monoclonal antibodies that block PD-1 or PD-L1, including Nivolumab (PD-1, BMS), Atezolizumab (PD-L1, Roche), Pembrolizumab (PD-1, Merck), Durvalumab (PD-L1, AZ), and Avelumab (PD-L1, Merck). These monoclonal antibodies have shown effective anti-tumor activity against a variety of malignancies.

Despite the favorable therapeutic outcomes achieved with such therapeutic agents, PD-1 monoclonal antibodies continue to present multiple challenges, including: 1) their large molecular size (160-170kD), which impairs their ability to penetrate solid tumors and traverse the blood-brain barrier, and 2) the substantial workload involved in the in vitro eukaryotic expression and cell-based screening, resulting in elevated manufacturing costs.

### Summary

Accordingly, the present invention provides an anti-PD-1 antibody (e.g., nanobody) and biological materials and products thereof. The anti-PD-1 antibody has one of the following advantages: small molecular weight, simpler structure, low immunogenicity, high tissue penetration, high stability, high solubility and low aggregation propensity or ease of cloning, and thus can effectively block the binding of PD-1 to PD-L1, and be used to prevent and/or treat PD-1 positive cancers.

The present invention provides the following technical solutions:
The present invention provides an anti-PD-1 antibody, a variant thereof, or an antigen-binding fragment thereof, wherein the anti-PD-1 antibody comprises three complementarity-determining regions CDR1, CDR2 and CDR3 (as indicated by underlined italics) contained in VHHs designated PD1-A1, PD1-A2, PD1-A3, PD1-C1, PD1-C2, PD1-C3, PD1-C4, PD1-C5, and PD1-C6.

| Unique ID | VHH amino acid sequences |
|---|---|
| PD1-A1 | QVQLVESGGGLVQAGGSLRLTCAATGTGADDYAIGWFR QVPGVEREGVSCIAKTDGSTFYAHFAKDRFTISRDNAKNA VYLQMNSLKPEDTGVYYCALLHDCHPAGTRVSLTGSWG PGTRVTVSS |
| PD1-A2 | QVQLVESGGGVVQVGGSLRLSCTASGNDFSIELMAWYR QAPGKEREWVAGISADTQNVEDSLEGRFTISRDSTKNTVY LQMNSLKPEDTALYRCNVRGYNMRNPNVGRLPYDAWGQ GTQVTVSS |
| PD1-A3 | QVQLVESGGGLVQAGGSLRLSCTAGGFKFDDYAVGWVR QAPGKEREGVACIEKSPNVDTYYAIFAKDRFTISRNDAQTT VYLQMNSLKVEDTALYHCAMLHYCPNAGTRVSLKGSWG PGTQVTVSS |
| PD1-C1 | QVKLVESGGGSVQAGGSLRLSCARANFASSPKTMAWFR QAPGKEREGVASIYTTTGITYYTDSVQGRFSISQDMAKNTV YLQMNSLKPEDTAMYYCAAALTEIGRYRLYARQYDFWG QGTQVTVSS |
| PD1-C2 | QVKLVESGGGSVQAGGSLRLSCAAPGYLYSNNCMGWF RQAPGKEREWVVGIHTGRPSQQSYADSVKDRFTISEDSAK NTVYLQMNSLKPEDTAMYYCAARPGGQKYDCYSGSWYR SGYWGQGTQVTVSS |
| PD1-C3 | QVQLVQSGGGLVQPGGSLRLSCAASGFTFSSYAMTWVR QTPGKGLEWVAAISSGDGVFTEYADSVKGRFTISRDNAKN TLYLQLNSLKTEDTAMYYCAKGGYETYDGRGQGTQVTV SS |
| PD1-C4 | EVKLVESGGGSVNTGGSLRLSCTSSGFTFDDFDVGWYR QVRRDECELVSTISSDGRTYYEDSVKGRFDISQDNAKNTV YLQMNRLAPEDAAVYYCAANPHFIAMSGPCTVVRRGMD YWGKGTLVTISS |
| PD1-C5 | QVQLVESGGGLVQPGGSLRLSCAASGFTFSSYAMTWVRQ TPGKGLEWVAAISSGDGVFTEYADSVKGRFTISRDNAKNT LYLQLNSLKTEDTAMYYCARLGTRGWYLDWGQGTQVT VSS |
| PD1-C6 | EVQLVESGGGSVEAGGSLTLSCLASGMNFDAADMGWY RQLPGNECEGVAAILTGVGTIYYADSLKGRFTISQDNAKNT VYLQMNSLKPEDTAMYYCAARPFGCNWRFYEYNYWGQ GTQVTVSS |

Exemplarily, for the antibodies and variants thereof described herein, or antigen-binding fragments thereof, the variants are humanized variants or variants having a sequence identity of at least 80%, 81%, 82%, 83%, 84%, 85%, 86%, 87%, 88%, 89%, 90%, 91%, 92%, 93%, 94%, 95%, 96%, 97%, 98%, 99%, 99.5%, or 99.9%.

Further, the antibodies and variants thereof, or antigen-binding fragments thereof, are selected from the group consisting of camel Ig, Ig NAR, Fab fragment, Fab' fragment, F(ab)'2 fragment, F(ab)'3 fragment, Fv, scFv, bi-scFv, (scFv)2 , minibodies, double-chain antibodies(diabodies), three-chain antibodies(triabodies), four-chain antibodies(tetrabodies), disulfide-stabilized Fv proteins, and single-domain antibodies (sdAb, nanoantibodies), bispecific antibodies, or trispecific antibodies.

The present invention also provides a fusion protein comprising the antibody and variants thereof, or an antigen-binding fragment thereof.

Exemplarily, the fusion protein described herein further comprises a tag sequence (e.g., Poly-His, hemagglutinin, c-Myc, GST, Flag-tag, etc.) or an IgG1 Fc protein sequence, or an additional epitope (e.g., another epitope directed against or different from PD-1) or an additional antibody active fragment (e.g., antibodies or antibody active fragments directed against other epitopes or the same epitope of PD-1, or ligands capable of binding to PD-1).

The present invention also provides an antibody-drug conjugate, which comprises the antibody and variants thereof, or an antigen-binding fragment thereof.

For the antibody-drug conjugates described herein, the drug is selected from the group consisting of: a radioactive label, ³²P, ³⁵S, a fluorescent dye, an electron-dense reagent, an enzyme, biotin, streptavidin, digoxigenin, a hapten, an immunogenic protein, a nucleic acid molecule having a sequence complementary to a target, or any combination of the foregoing; or an immunomodulatory compound, an anticancer agent, an antiviral agent, an antibacterial agent, an antifungal agent or an antiparasitic agent, or any combination of the foregoing.

The present invention also provides an isolated polynucleotide encoding an antibody or an antigen-binding fragment thereof, characterized in that the polynucleotide is capable of expressing the antibody, variants thereof, or its antigen-binding fragment described herein; the polynucleotide is capable of expressing the fusion protein described herein; or the polynucleotide is capable of expressing the antibody-drug conjugate described herein.

The present invention also provides a vector comprising the polynucleotide described herein, preferably a plasmid vector.

The present invention also provides a host cell, which comprises the polynucleotide described herein or the vector described herein. Preferably, the host cell is a eukaryotic cell.

The present invention also provides a pharmaceutical composition comprising the antibody and variants thereof described herein, or antigen-binding fragments thereof, the fusion protein described herein, the antibody-drug conjugate described herein, and optionally, a pharmaceutically acceptable carrier.

The present invention also provides use of the antibody and variants thereof described herein, or antigen-binding fragments thereof, the fusion protein described herein, and the antibody-drug conjugate described herein in the manufacture of a medicament for treating and/or preventing diseases associated with PD-1.

The present invention also provides a method for treating and/or preventing PD-1-associated diseases and related symptoms, which comprises administering an effective amount of the antibody and variants thereof described herein, or an antigen-binding fragment thereof, the fusion protein described herein, the antibody-drug conjugate described herein, or the pharmaceutical composition described herein to a subject.

The present invention also provides a method for detecting whether a sample contains T cells that highly express PD-1, which comprises the step of contacting the antibody and variants thereof described herein, or antigen-binding fragments thereof, or the fusion protein described herein with the sample. Optionally, the detection may be for diagnostic purposes or non-diagnostic purposes.

The present invention also provides a detection product, which comprises the antibody and variants thereof described herein, or an antigen-binding fragment thereof.

Illustratively, for the detection product described herein, the product is selected from one or more of a detection reagent, a kit, a chip or a test strip.

In order to achieve the foregoing object of the invention, the present invention further provides the following technical solutions:
In a first aspect, the present invention provides an anti-PD-1 antibody (e.g., a nanobody), wherein the heavy chain variable region of the anti-PD-1 antibody (e.g., a nanobody) consists of a framework region FR and a complementarity-determining region (CDR), and the complementarity-determining region (CDR) comprises any one or at least one of the following groups:
(1) CDR1 as set forth in SEQ ID NO: 1, CDR2 as set forth in SEQ ID NO: 2, and CDR3 as set forth in SEQ ID NO: 3;
(2) CDR1 as set forth in SEQ ID NO: 8, CDR2 as set forth in SEQ ID NO: 9, and CDR3 as set forth in SEQ ID NO: 10;
(3) CDR1 as set forth in SEQ ID NO: 15, CDR2 as set forth in SEQ ID NO: 16, and CDR3 as set forth in SEQ ID NO: 17;
(4) CDR1 as set forth in SEQ ID NO: 22, CDR2 as set forth in SEQ ID NO: 23, and CDR3 as set forth in SEQ ID NO: 24;
(5) CDR1 as set forth in SEQ ID NO: 29, CDR2 as set forth in SEQ ID NO: 30, and CDR3 as set forth in SEQ ID NO: 31;
(6) CDR1 as set forth in SEQ ID NO: 36, CDR2 as set forth in SEQ ID NO: 37, and CDR3 as set forth in SEQ ID NO: 38;
(7) CDR1 as set forth in SEQ ID NO: 43, CDR2 as set forth in SEQ ID NO: 44, and CDR3 as set forth in SEQ ID NO: 45;
(8) CDR1 as set forth in SEQ ID NO: 50, CDR2 as set forth in SEQ ID NO: 51, and CDR3 as set forth in SEQ ID NO: 52;
(9) CDR1 as set forth in SEQ ID NO: 57, CDR2 as set forth in SEQ ID NO: 58 and CDR3 as set forth in SEQ ID NO: 59.

Nanobodies (Nbs) are the variable domains of heavy-chain antibodies (IgG2 and IgG3) in camelids, and represent the smallest naturally occurring antigen-binding fragments. Compared with conventional full-length monoclonal antibodies (mAbs, about 150kDa), Nb has the advantages of small molecular weight (12-15KD), simpler structure, low immunogenicity, high tissue permeability, high stability, high solubility, low aggregation propensity, and ease of cloning. In addition, compared with mAb counterparts, the production cost of Nb is significantly reduced, and affordable for most cancer patients. In September 2018, the European Medicines Agency approved the first nanobody drug caplacizumab (trade name: Cablivi), which is mainly used to treat acquired thrombotic thrombocytopenic purpura (aTTP) in adults. Therefore, Nbs are expected to provide a wide range of application scenarios in cancer treatment and diagnosis.

In the present study, camels were immunized with recombinant PD-1 antigens to construct a VHH phage library, and nine functional anti-PD-1 Nbs were obtained after biopanning. For example, by comparing the blocking effects of Nbs on PD-1 and PD-L1, Nbs designated PD1-A2, PD1-C1, and PD1-C6, respectively, can be used to block the PD-1/PD-L1 signaling pathway.

In the embodiment provided by the present invention, the framework region FR comprises any one or at least one of the following groups:
(1) FR1 as set forth in SEQ ID NO: 4, FR2 as set forth in SEQ ID NO: 5, FR3 as set forth in SEQ ID NO: 6, and FR4 as set forth in SEQ ID NO: 7;
(2) FR1 as set forth in SEQ ID NO: 11, FR2 as set forth in SEQ ID NO: 12, FR3 as set forth in SEQ ID NO: 13, and FR4 as set forth in SEQ ID NO: 14;
(3) FR1 as set forth in SEQ ID NO: 18, FR2 as set forth in SEQ ID NO: 19, FR3 as set forth in SEQ ID NO: 20, and FR4 as set forth in SEQ ID NO: 21;
(4) FR1 as set forth in SEQ ID NO: 25, FR2 as set forth in SEQ ID NO: 26, FR3 as set forth in SEQ ID NO: 27, and FR4 as set forth in SEQ ID NO: 28;
(5) FR1 as set forth in SEQ ID NO:32, FR2 as set forth in SEQ ID NO:33, FR3 as set forth in SEQ ID NO:34, and FR4 as set forth in SEQ ID NO:35;
(6) FR1 as set forth in SEQ ID NO:39, FR2 as set forth in SEQ ID NO:40, FR3 as set forth in SEQ ID NO:41, and FR4 as set forth in SEQ ID NO:42;
(7) FR1 as set forth in SEQ ID NO:46, FR2 as set forth in SEQ ID NO:47, FR3 as set forth in SEQ ID NO:48, and FR4 as set forth in SEQ ID NO:49;
(8) FR1 as set forth in SEQ ID NO:53, FR2 as set forth in SEQ ID NO:54, FR3 as set forth in SEQ ID NO:55, and FR4 as set forth in SEQ ID NO:56; or
(9) FR1 as set forth in SEQ ID NO:60, FR2 as set forth in SEQ ID NO:61, FR3 as set forth in SEQ ID NO:62, and FR4 as set forth in SEQ ID NO:63.

However, the framework region FR of the present invention is not limited to the above sequences, and any sequence that can achieve its function is within the protection scope of the present invention.

In the embodiments provided by the present invention, the amino acid sequence of the anti-PD-1 nanobody is as set forth in SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71 or SEQ ID NO: 72.

The CDR sequences and FR sequences of PD1-A2, PD1-C1, and PD1-C6 are as follows:

| Identifier | Regions | Sequences | SEQ Nos |
|---|---|---|---|
| PD1-A2 | CDR1 | IELMA | 1 |
| | CDR2 | GISADTQNVEDSLEG | 2 |
| | CDR3 | RGYNMRNPNVGRLPYDA | 3 |
| | FR1 | QVQLVESGGGVVQVGGSLRLSCTASGNDFS | 4 |
| | FR2 | WYRQAPGKEREWVA | 5 |
| | FR3 | RFTISRDSTKNTVYLQMNSLKPEDTALYRCNV | 6 |
| | FR4 | WGQGTQVTVSS | 7 |
| PD1-C1 | CDR1 | PKTMA | 8 |
| | CDR2 | SIYTTTGITYYTDSVQG | 9 |
| | CDR3 | ALTEIGRYRLYARQYDF | 10 |
| | FR1 | QVKLVESGGGSVQAGGSLRLSCARANFASS | 11 |
| | FR2 | WFRQAPGKEREGVA | 12 |
| | FR3 | RFSISQDMAKNTVYLQMNSLKPEDTAMYYCAA | 13 |
| | FR4 | WGQGTQVTVSS | 14 |
| PD1-C6 | CDR1 | AADMG | 15 |
| | CDR2 | AILTGVGTIYYADSLKG | 16 |
| | CDR3 | RPFGCNWRFYEYNY | 17 |
| | FR1 | EVQLVESGGGSVEAGGSLTLSCLASGMNFD | 18 |
| | FR2 | WYRQLPGNECEGVA | 19 |
| | FR3 | RFTISQDNAKNTVYLQMNSLKPEDTAMYYCAA | 20 |
| | FR4 | WGQGTQVTVSS | 21 |

The CDR sequences and FR sequences of PD1-A1, PD1-A3, and PD1-C2 to C5 are as
follows:

| Identifier | Regions | Sequences | SEQ Nos |
|---|---|---|---|
| PD1-A1 | CDR1 | DYAIG | 22 |
| | CDR2 | CIAKTDGSTFYAHFAKD | 23 |
| | CDR3 | LHDCHPAGTRVSLTGS | 24 |
| | FR1 | QVQLVESGGGLVQAGGSLRLTCAATGTGAD | 25 |
| | FR2 | WFRQVPGVEREGVS | 26 |
| | FR3 | RFTISRDNAKNAVYLQMNSLKPEDTGVYYCAL | 27 |
| | FR4 | WGPGTRVTVSS | 28 |
| PD1-A3 | CDR1 | DYAVG | 29 |
| | CDR2 | CIEKSPNVDTYYAIFAKD | 30 |
| | CDR3 | LHYCPNAGTRVSLKGS | 31 |
| | FR1 | QVQLVESGGGLVQAGGSLRLSCTAGGFKFD | 32 |
| | FR2 | WVRQAPGKEREGVA | 33 |
| | FR3 | RFTISRNDAQTTVYLQMNSLKVEDTALYHCAM | 34 |
| | FR4 | WGPGTQVTVSS | 35 |
| PD1-C2 | CDR1 | NNCMG | 36 |
| | CDR2 | GIHTGRPSQQSYADSVKD | 37 |
| | CDR3 | RPGGQKYDCYSGSWYRSGY | 38 |
| | FR1 | QVKLVESGGGSVQAGGSLRLSCAAPGYLYS | 39 |
| | FR2 | WFRQAPGKEREWVV | 40 |
| | FR3 | RFTISEDSAKNTVYLQMNSLKPEDTAMYYCAA | 41 |
| | FR4 | WGQGTQVTVSS | 42 |
| PD1-C3 | CDR1 | SYAMT | 43 |
| | CDR2 | AISSGDGVFTEYADSVKG | 44 |
| | CDR3 | GGYETYDG | 45 |
| | FR1 | QVQLVQSGGGLVQPGGSLRLSCAASGFTFS | 46 |
| | FR2 | WVRQTPGKGLEWVA | 47 |
| | FR3 | RFTISRDNAKNTLYLQLNSLKTEDTAMYYCAK | 48 |
| | FR4 | RGQGTQVTVSS | 49 |
| PD1-C4 | CDR1 | DFDVG | 50 |
| | CDR2 | TISSDGRTYYEDSVKG | 51 |
| | CDR3 | NPHFIAMSGPCTVVRRGMDY | 52 |
| | FR1 | EVKLVESGGGSVNTGGSLRLSCTSSGFTFD | 53 |
| | FR2 | WYRQVRRDECELVS | 54 |
| | FR3 | RFDISQDNAKNTVYLQMNRLAPEDAAVYYCAA | 55 |
| | FR4 | WGKGTLVTISS | 56 |
| PD1-C5 | CDR1 | SYAMT | 57 |
| | CDR2 | AISSGDGVFTEYADSVKG | 58 |
| | CDR3 | LGTRGWYLD | 59 |
| | FR1 | QVQLVESGGGLVQPGGSLRLSCAASGFTFS | 60 |
| | FR2 | WVRQTPGKGLEWVA | 61 |
| | FR3 | RFTISRDNAKNTLYLQLNSLKTEDTAMYYCAR | 62 |
| | FR4 | WGQGTQVTVSS | 63 |

SEQ ID NO:64(PD1-A1): 
QVQLVESGGGLVQAGGSLRLTCAATGTGADDYAIGWFRQVPGVEREGVSCIAK
TDGSTFYAHFAKDRFTISRDNAKNAVYLQMNSLKPEDTGVYYCALLHDCHPAGTRV
SLTGSWGPGTRVTVSS
SEQ ID NO:65(PD1-A2): 
QVQLVESGGGVVQVGGSLRLSCTASGNDFSIELMAWYRQAPGKEREWVAGISA
DTQNVEDSLEGRFTISRDSTKNTVYLQMNSLKPEDTALYRCNVRGYNMRNPNVGRL
 PYDAWGQGTQVTVSS
SEQ ID NO:66(PD1-A3): 
QVQLVESGGGLVQAGGSLRLSCTAGGFKFDDYAVGWVRQAPGKEREGVACIE
KSPNVDTYYAIFAKDRFTISRNDAQTTVYLQMNSLKVEDTALYHCAMLHYCPNAGT
RVSLKGSWGPGTQVTVSS
SEQ ID NO:67(PD1-C1): 
QVKLVESGGGSVQAGGSLRLSCARANFASSPKTMAWFRQAPGKEREGVASIYT
TTGITYYTDSVQGRFSISQDMAKNTVYLQMNSLKPEDTAMYYCAAALTEIGRYRLY
ARQYDFWGQGTQVTVSS
SEQ ID NO:68(PD1-C2): 
QVKLVESGGGSVQAGGSLRLSCAAPGYLYSNNCMGWFRQAPGKEREWVVGIH
TGRPSQQSYADSVKDRFTISEDSAKNTVYLQMNSLKPEDTAMYYCAARPGGQKYDC
YSGSWYRSGYWGQGTQVTVSS
SEQ ID NO:69(PD1-C3): 
QVQLVQSGGGLVQPGGSLRLSCAASGFTFSSYAMTWVRQTPGKGLEWVAAISS
GDGVFTEYADSVKGRFTISRDNAKNTLYLQLNSLKTEDTAMYYCAKGGYETYDGRG
QGTQVTVSS
SEQ ID NO:70(PD1-C4): 
EVKLVESGGGSVNTGGSLRLSCTSSGFTFDDFDVGWYRQVRRDECELVSTISSD
GRTYYEDSVKGRFDISQDNAKNTVYLQMNRLAPEDAAVYYCAANPHFIAMSGPCTV
VRRGMDYWGKGTLVTISS
SEQ ID NO:71(PD1-C5): 
QVQLVESGGGLVQPGGSLRLSCAASGFTFSSYAMTWVRQTPGKGLEWVAAISS
GDGVFTEYADSVKGRFTISRDNAKNTLYLQLNSLKTEDTAMYYCARLGTRGWYLD
WGQGTQVTVSS
SEQ ID NO:72(PD1-C6):

EVQLVESGGGSVEAGGSLTLSCLASGMNFDAADMGWYRQLPGNECEGVAAILT
GVGTIYYADSLKGRFTISQDNAKNTVYLQMNSLKPEDTAMYYCAARPFGCNWRFYE
YNYWGQGTQVTVSS

The second aspect of the present invention provides a polynucleotide, which is a polynucleotide encoding the foregoing anti-PD-1 antibody (e.g., a nanobody).

The third aspect of the present invention provides a recombinant expression vector, which comprises the foregoing polynucleotide.

In the embodiments provided by the present invention, the expression vector comprises a prokaryotic expression vector or a eukaryotic expression vector.

The fourth aspect of the present invention provides a recombinant host cell, which comprises the foregoing polynucleotide, or comprises the foregoing recombinant expression vector.

In the embodiments provided by the present invention, the host cell includes a prokaryotic cell or a eukaryotic cell.

In a specific embodiment provided by the present invention, the host cell is selected from Escherichia coli or yeast cells.

In a specific embodiment provided by the present invention, the host cell is selected from HEK293T cells, HEK293F cells, Expi293F cells or CHO cells.

A fifth aspect of the present invention provides a method for preparing the foregoing anti-PD-1 antibody (e.g., nanobody), comprising the following steps:
inserting a polynucleotide encoding the foregoing anti-PD-1 antibody (e.g., nanobody) into an expression vector to obtain a recombinant expression vector;
introducing the recombinant expression vector into a host cell to obtain a recombinant host cell;
culturing the recombinant host cell to obtain a culture; and
purifying the culture to obtain the anti-PD-1 antibody (eg, nanobodies).

In the embodiments provided by the present invention, purification is performed using Protein A agarose resin.

A sixth aspect of the present invention provides a bispecific antibody, which comprises the foregoing anti-PD-1 antibody (eg, nanobody) and a second antibody.

In the embodiments provided by the present invention, the second antibody includes, but is not limited to, 4-1BB nanobody, CD47 nanobody, VEGF nanobody, HER2 nanobody, EGFR nanobody, HER3 nanobody, B7H3 nanobody, TIGIT nanobody, OX-40 nanobody, CD40 nanobody or PD-L1 nanobody.

A seventh aspect of the present invention provides the use of the foregoing anti-PD-1 antibodies (e.g., nanobody) or bispecific antibodies in the manufacture of a medicament for preventing and/or treating cancer and detecting PD-1 proteins.

In the embodiments provided by the present invention, cancer includes, but is not limited to, gastric cancer, liver cancer, leukemia, renal tumor, lung cancer, small intestine cancer, bone cancer, prostate cancer, colorectal cancer, breast cancer, large intestine cancer, prostate cancer, cervical cancer, lymphoma, adrenal tumor or bladder tumor.

An eighth aspect of the present invention provides a pharmaceutical composition, comprising: the foregoing anti-PD-1 antibody (e.g., nanobody) or bispecific antibody; and pharmaceutically acceptable excipients.

In the embodiments provided by the present invention, the dosage form of the pharmaceutical composition includes, but is not limited to, injection, powder for injection, tablet or capsule.

A ninth aspect of the present invention provides a kit for detecting PD-1 protein, the kit comprising: the foregoing anti-PD-1 antibody (eg, nanobody) or bispecific antibody; and a detection-acceptable reagent.

The tenth aspect of the present invention provides a chimeric antigen receptor (CAR), the extracellular domain of which comprises the anti-PD-1 antibody described herein.

The eleventh aspect of the present invention provides a cell comprising or expressing the CAR described herein, such as a T cell or a NK cell.

A twelfth aspect of the present invention provides a method for treating cancer, which comprises administering to a subject the antibody described herein or a cell comprising or expressing the CAR described herein, such as a T cell or a NK cell.

Compared with the prior art, the present invention has one of the following beneficial effects: As demonstrated by affinity assays between the antibody (e.g., nanobody) and recombinant antigen protein, the VHH-his recombinant antibodies of PD1-A2, PD1-C1, and PD1-C6 bind to the recombinant extracellular domain protein of human PD-1 with an EC₅₀ of 0.13-42.17 µg/mL;
As demonstrated by affinity assays between the antibody (e.g., nanobody) and recombinant antigen protein, the VHH-his recombinant antibodies of PD1-A2, PD1-C1, and PD1-C6 can bind to 293F cells overexpressing human PD-1;
As demonstrated by PD-1/PD-L1 blocking assays, compared with the positive control drug Keytruda (full-length IgG antibody), PD1-A2, PD1-C1, and PD1-C6 (VHH-His recombinant antibody) can block the binding of PD-1 to PD-L1 and restore the TCR signaling pathway; further, PD1-C1 recombinant antibody completely blocks the binding of PD-1 to PD-L1 with an IC₅₀ of 0.68 µg/mL, while Keytruda exhibits an IC₅₀ of 0.57 µg/mL.

### BRIEF DESCRIPTION OF THE DRAWINGS

FIG. 1 is an ELISA binding curve of VHH-His recombinant antibody to human PD-1 (hFc tag);
FIG. 2 is a dose-response curve of PD-1 antibody blocking PD-1/PD-L1 binding;
FIG. 3 is an ELISA binding curve of VHH-His recombinant antibody and monkey PD-1 (His tag).

### DETAILED DESCRIPTION

The present invention discloses an anti-PD-1 antibody (e.g., nanobody) and biological materials and products thereof. Those skilled in the art may refer to the contents herein and appropriately adjust process parameters to achieve it. It should be noted in particular that all similar substitutions and modifications are obvious to those skilled in the art and shall be deemed to be encompassed by the present invention. The methods and applications of the present invention have been described in preferred embodiments. Those skilled in the art may readily make modifications, alterations or combinations of the methods and applications described herein without departing from the spirit, content and scope of the present invention, so as to implement and apply the technical solutions of the present invention.

The present invention will be further illustrated with reference to the following examples.

### Example 1: Antigen preparation and animal immunization

### 1. Animal immunization:

Recombinant protein of human PD-1 extracellular domain (with hFc tag) was administered via multi-site subcutaneous and intramuscular injection at the neck and dorsal region of one alpaca and one Bactrian camel (*Camelus bactrianus*) to form multiple local nodules. The absorption of subcutaneous nodules was monitored to verify successful immunization.

For the primary immunization, 0.5 mg of antigen was mixed with Freund's complete adjuvant in a ratio of 1:1 and injected after emulsification, with a volume of 1 mL per alpaca or camel;

For the second immunization: 3 weeks after the primary immunization, 0.25 mg of antigen was mixed with Freund's incomplete adjuvant in a ratio of 1:1, emulsified and injected, with an injection volume of 1 mL per alpaca or camel;

For the third immunization: 3 weeks after the second immunization, 0.25 mg of antigen was mixed with Freund's incomplete adjuvant in a ratio of 1:1, emulsified and injected, with an injection volume of 1 mL per alpaca or camel;

For the fourth immunization: three weeks after the third immunization, 0.25 mg antigen was mixed with Freund's incomplete adjuvant at a ratio of 1:1, emulsified and injected at 1 mL per animal.

### 2. Serum processing and titer detection:

One week after the third and fourth immunizations, 2 mL of peripheral blood was collected, and serum was isolated. Recombinant protein of human PD-1 extracellular domain (with His tag) was coated on 96-well ELISA plates, and serum antibody titers were determined by ELISA.

The ELISA results demonstrated that the serum titer of the alpaca after three rounds of immunization was higher than 1:64,000, meeting the criteria for library establishment; the serum titer of the Bactrian camel after four rounds of immunization was higher than 1:64,000, which also met the library preparation criteria.

### Example 2: Construction of phage display immune antibody library

Since the serum titer of the alpaca after the third immunization was >1:32000, and the serum titer of the Bactrian camel after the fourth immunization was >1:32000, it indicated that high-affinity antibodies against human PD-1 were present in the serum. Therefore, the phage display immune antibody library was constructed according to the following steps:
① Collect 50 mL of peripheral blood from the third immunization of the alpaca and the fourth immunization of the Bactrian camel, and separate PBMCs; take 2×10⁷ PBMCs, and extract total RNA with an RNA extraction kit; take an appropriate amount of RNA (such as 3-5 µg) and synthesize cDNA using an RT-PCR reverse transcription kit.
② The IgG2 and IgG3 heavy chain variable region sequences (the heavy chain variable region VHH of the nanobody) were obtained step by step by nested PCR. The experimental steps are as follows:
   1) design a pair of specific nested outer primers, and perform the first round of PCR amplification with cDNA as the template. The amplified region corresponds to the Leader-CH2 region of the heavy chain antibody gene of alpaca and Bactrian camel, with product sizes of 700bp and 900bp; perform DNA gel electrophoresis, and recover the 700 bp PCR product by gel extraction; 2) design nested inner primers (3 pairs for alpaca and 5 pairs for Bactrian camel), and perform the second round of PCR amplification using the 700 bp product of the first round PCR as the template. The amplified region is the VHH fragment of the heavy chain antibody variable region of alpaca and Bactrian camel, with a product size of 400 bp; purify and recover the product of the second round PCR using a PCR product purification kit.
③ Insert the heavy chain variable region sequence into the linearized phagemid vector VHH-lib Template digested with restriction enzymes through homologous recombination or restriction enzyme ligation to obtain the recombinant vector; after purification and recovery, transform the super-competent SS320 cells (harboring the helper phage M13K07); resuspend the transformed bacterial solution in SOC medium and incubate for 1 hour for activation; take a small amount of bacterial solution for 10-fold gradient dilution, select the appropriate dilution titer, spread it on LB/tet10 and LB/Carb50 culture plates, incubate overnight in a 37°C biochemical incubator, and use the plates for library titer calculation on the next day; transfer the remaining bacterial solution into a large volume of 2YT/Carb50/Kan25 liquid culture medium, culture overnight in a 37°C shaker, collect the supernatant the next day, add 1/4 volume of PEG/NaCl solution to precipitate phages, resuspend the precipitated phages with an appropriate amount of PBT solution and dilute to the required concentration, so as to obtain the phage display immune antibody library (stored at -80°C for future use).
④ Count the number of clones on the LB/Carb50 plate and calculate the library size or capacity: the library size or capacity of the alpaca antibody library Lib PD-1 Alpaca is 1.78×10⁹, and the library size or capacity of the camel antibody library Lib PD-1 Camel is 4.6×10⁹. Twenty single colonies were randomly selected from each plate for sequencing, and the results show that the VHH insertion efficiency of the alpaca antibody library Lib PD-1 Alpaca is 75%, and the VHH insertion efficiency of the camel antibody library Lib PD-1 Camel is 80%.

### Example 3: Screening of antibody library

5 µg/mL of human PD-1 extracellular domain recombinant protein (His tag) was added to a 96-well plate (100 µL/well) and coated overnight at 4°C; Escherichia coli strain NEB5αF' was streaked on a 2YT/Tet10 plate and cultured overnight at 37°C; on the next day, single colonies of NEB5αF' were picked from the overnight 2YT/Tet10 plate and inoculated into 3 mL of 2YT/Tet10 liquid culture medium, and cultured with shaking at 37°C until OD600 reached 0.8; at the same time, the antigen coating solution of the 96-well plate was discarded. Each well was blocked with 200 µL of 1% BSA, and blank wells were added with 200 µL of 1% BSA as negative controls.

The plate was incubated on a 3D rotating shaker at room temperature for 2 hours. Afterwards, the supernatant of antigen wells and control wells was discarded, and each well was washed twice with 200 µL of PT buffer. **Subsequently,** 100 µL of the phage antibody library was added to each well, and incubated on a 3D rotating oscillator at room temperature for 2 hours. The supernatant was removed, and the wells were washed with 200 µL of PT buffer. Then, 100 µL of 100 mM HCl was added for elution, and incubated at room temperature for 5 minutes. The eluted supernatant was transferred into a 1.5 mL centrifuge tube and neutralized with 1 M Tris-HCl.

The neutralized mixture was added to a centrifuge tube containing 1 mL of NEB5αF' bacterial solution, and cultured with shaking at 37 °C for 1 hour. A 20 µL aliquot of the bacterial culture was serially diluted appropriately and spread on LB/Carb50 plates, which were incubated overnight in a 37 °C biochemical incubator for titer and enrichment evaluation on the following day. To the remaining culture, 1 µL of helper phage M13K07 (final concentration: 1×10¹⁰ CFU/mL) was added, and the mixture was cultured with shaking at 37 °C for 1 hour. The resulting culture was transferred into 35 mL of 2YT/Carb50/Kan25 liquid medium and cultured overnight at 37 °C with shaking. The amplified phages were harvested to obtain the antibody library for the next round of panning.

The above operations were repeated for 2-3 rounds until obvious phage enrichment was observed. Enrichment was regarded as successful when the colony number of antigen-binding wells was more than 10-fold higher than that of negative control wells on LB/Carb50 plates. In this experiment, for Lib PD-1 Alpaca, the colony number of antigen-binding wells after the third round of screening was 100 times that of the negative control wells; for Lib PD-1 Camel, the colony number after the second round of screening reached 10 times that of the negative control wells, confirming successful enrichment.

Clones obtained after enrichment rounds were randomly picked and cultured in 96-deep-well plates for expansion. After centrifugation, the culture supernatants were collected for phage ELISA screening. Positive clones were defined as those with a ratio of the OD value for human PD-1 extracellular domain recombinant protein (His tag) binding to the OD value for blocking solution binding greater than 2. The identified positive clones were subjected to sequencing and sequence alignment to obtain unique VHH sequences.

### Example 4: NGS sequencing

Primers were designed to target the constant regions flanking both sides of the VHH. The variable regions of the phage library were amplified. The PCR products were analyzed via E-gel electrophoresis, and the PCR library fragments were purified and recovered using a commercial kit for NGS sequencing. The obtained data were statistically analyzed with SPSS 2.0 and Microsoft Excel 2019, and sorted according to DNA sequence counts and frequency.

### Example 5: Alignment and Statistical Analysis of Positive Sequences and NGS High-Frequency Sequences

Phage ELISA positive sequences and high-frequency NGS sequences (Top 100-500) were subjected to sequence alignment and grouped based on the differences in CDR H3 regions. The results are as set forth in Table 1 and Table 2.

**Table 1. PD-1 nanoantibodies obtained from alpaca immunization**

| **Group** | **Unique ID** | **VHH amino acid sequence** |
|---|---|---|
| 1 | PD1-A1 | QVQLVESGGGLVQAGGSLRLTCAATGTGADDYAIGWFRQ VPGVEREGVSCIAKTDGSTFYAHFAKDRFTISRDNAKNAVY LQMNSLKPEDTGVYYCALLHDCHPAGTRVSLTGSWGPGT RVTVSS |
| 2 | PD1-A2 | QVQLVESGGGVVQVGGSLRLSCTASGNDFS*IELMA*WYRQ APGKEREWVA*GISADTQNVEDSLEG*RFTISRDSTKNTVYLQ MNSLKPEDTALYRCNV*RGYNMRNPNVGRLPYDA*WGQGTQ VTVSS |
| 3 | PD1-A3 | QVQLVESGGGLVQAGGSLRLSCTAGGFKFDDYAVGWVR QAPGKEREGVACIEKSPNVDTYYAIFAKDRFTISRNDAQTT VYLQMNSLKVEDTALYHCAMLHYCPNAGTRVSLKGSWGP GTOVTVSS |

| | | |
|---|---|---|
| Note: The underlined part is the CDR region. | | |

**Table 2. PD-1 Nanobodies Obtained from Camel Immunization**

| **Group** | **Unique ID** | **VHH amino acid sequence** |
|---|---|---|
| 1 | PD1-C1 | QVKLVESGGGSVQAGGSLRLSCARANFASSPKTMA WFRQAPGKEREGVASIYTTTGITYYTDSVQGRFSISQD MAKNTVYLQMNSLKPEDTAMYYCAAALTEIGRYRLY ARQYDFWGQGTQVTVSS |
| 2 | PD1-C2 | QVKLVESGGGSVQAGGSLRLSCAAPGYLYSNNCMG WFRQAPGKEREWVVGIHTGRPSQQSYADSVKDRFTIS EDSAKNTVYLQMNSLKPEDTAMYYCAARPGGQKY DCYSGSWYRSGYWGQGTQVTVSS |
| 3 | PD1-C3 | QVQLVQSGGGLVQPGGSLRLSCAASGFTFSSYAMTW VRQTPGKGLEWVAAISSGDGVFTEYADSVKGRFTISR DNAKNTLYLQLNSLKTEDTAMYYCAKGGYETYDGR GQGTQVTVSS |
| 4 | PD1-C4 | EVKLVESGGGSVNTGGSLRLSCTSSGFTFDDFDVGW YRQVRRDECELVSTISSDGRTYYEDSVKGRFDISQDNA KNTVYLQMNRLAPEDAAVYYCAANPHFIAMSGPCT VVRRGMDYWGKGTLVTISS |
| 5 | PD1-C5 | QVQLVESGGGLVQPGGSLRLSCAASGFTFSSYAMTW VRQTPGKGLEWVAAISSGDGVFTEYADSVKGRFTISR DNAKNTLYLQLNSLKTEDTAMYYCARLGTRGWYLD WGQGTQVTVSS |
| 6 | PD1-C6 | EVQLVESGGGSVEAGGSLTLSCLASGMNFDAADMG WYRQLPGNECEGVAAILTGVGTIYYADSLKGRFTISQD NAKNTVYLQMNSLKPEDTAMYYCAARPFGCNWRF YEYNYWGQGTQVTVSS |

| | | |
|---|---|---|
| Note: The underlined part is the CDR region. | | |

### Example 6: Eukaryotic expression of nanobodies

A total of 9 sequences listed in Table 1 and Table 2 of Example 5 were subjected to eukaryotic expression. The experimental steps are as follows:
1) VHH fragments of the above sequences were amplified by PCR, and inserted into the His-tagged eukaryotic expression vector pFcIG-His via homologous recombination or restriction enzyme ligation. The constructed vectors were electrotransformed into *E. coli* Trans5α host cells. After screening with bleomycin, single clones were sequenced to verify correct recombinant plasmids. Subsequently, host cells harboring recombinant plasmids were expanded and cultured, and sterile endotoxin-free plasmids were extracted using an endotoxin removal kit.
2) HEK293F cells were cultured in serum-free medium. The recombinant expression plasmids were transfected into HEK293F cells using Polyplus suspension cell transfection reagent for protein expression. Supplemental feeding was performed at 24 hours and 72 hours post transfection. Cell culture supernatants were harvested on Day 5. Antibodies were isolated and purified by Protein A agarose purification resin, and buffer-exchanged and stored in PBS solution.

The experimental results (Table 3) showed that the transient expression yield of 9 recombinant VHH-His antibodies in HEK293F cells ranged from 5.4 to 36.4 mg/L. SDS-PAGE identification indicated that the VHH-His proteins encoded by PD1-A1, PD1-A2, PD1-A3, PD1-C1, PD1-C2 and PD1-C3 presented normal band sizes with purity > 95%. The recombinantly expressed antibodies of PD1-C4, PD1-C5 and PD1-C6 exhibited non-specific bands with purity < 95%.

**Table 3. Eukaryotic transient expression results of VHH-His recombinant antibodies**

| **Unique ID** | **Antibody type** | **Expression volume(mL)** | **Production(mg)** | **SDS-PAGE Purity** |
|---|---|---|---|---|
| PD1-A1 | VHH-his | 50 | 1.61 | >95% |
| PD1-A2 | VHH-his | 50 | 1.30 | >95% |
| PD1-A3 | VHH-his | 50 | 1.01 | >95% |
| PD1-C1 | VHH-his | 50 | 1.77 | >95% |
| PD1-C2 | VHH-his | 50 | 0.45 | >95% |
| PD1-C3 | VHH-his | 50 | 1.82 | >95% |
| PD1-C4 | VHH-his | 50 | 0.46 | >80% |
| PD1-C5 | VHH-his | 50 | 0.29 | >80% |
| PD1-C6 | VHH-his | 50 | 0.27 | >70% |

### Example 7: EC₅₀ Binding Affinity of Nanobodies against Recombinant Antigen Proteins

The binding affinity of 9 recombinant VHH-His antibodies to human PD-1 extracellular domain recombinant protein was determined by ELISA. Briefly, human PD-1 extracellular domain recombinant protein (hFc tag) was added to 96-well ELISA plates at 100 ng per well and coated overnight at 4 °C. The VHH-His recombinant antibodies were serially diluted to different concentrations (0.01-10 µg/mL) and incubated with the coated antigen. HRP-conjugated anti-His secondary antibodies were used for chromogenic reaction. The absorbance at 450 nm was measured with a microplate reader.

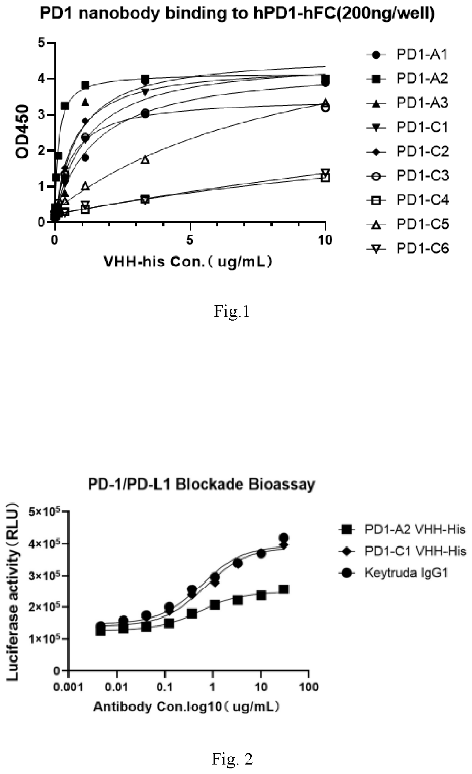

The experimental results (Table 4 and FIG. 1) indicated that the EC₅₀ values of VHH-His recombinant antibodies (PD1-A1, PD1-A2, PD1-A3, PD1-C1, PD1-C2, PD1-C3 and PD1-C5) binding to human PD-1 extracellular domain recombinant protein ranged from 0.13 to 9.86 µg/mL.

**Table 4. Binding affinity of VHH-His recombinant antibodies to human PD-1 (hFc tag)**

| Unique ID | Binding affinity to human PD-1 EC₅₀(µg/mL) |
|---|---|
| PD1-A1 | 1.47 |
| PD1-A2 | 0.13 |
| PD1-A3 | 0.78 |
| PD1-C1 | 1.13 |
| PD1-C2 | 0.67 |
| PD1-C3 | 0.59 |
| PD1-C4 | 23.00 |
| PD1-C5 | 9.86 |
| PD1-C6 | 42.17 |

### Example 8: Binding of Nanobodies to Human PD-1 Overexpressing Cells

Flow cytometry was used to detect the binding activity of nanobodies to human PD-1-overexpressing 293F cells:
1)293F cells were transfected with a eukaryotic expression plasmid carrying the full-length human PD-1 gene and continuously cultured for 24 hours;
2)Collect 0.3×10⁶ 293F cells transiently overexpressing human PD-1, wash the cells twice with PBS, and resuspend in 100 µL PBS;
3)Incubate the cells with VHH-His recombinant antibodies (10 µg/mL and 50 µg/mL) or PD-L1 (His-tagged) recombinant protein for 1 hour;
4)After washing the cells three times with PBS, resuspend the cells in 100 µL PBS, add 0.2 µg/mL PE-conjugated anti-His antibody, and incubate for 1 hour;
5)Following three PBS washes, the cells were resuspended in 300 µL PBS, and fluorescence signals were detected by a flow cytometer. The results are as set forth in Table 5.

The experimental results indicated that the VHH-His recombinant antibodies of PD1-A1, PD1-A2, PD1-C1, PD1-C2, PD1-C3 and PD1-C5 are capable of binding to human PD-1-overexpressing 293F cells.

**Table 5. Binding affinity of VHH-His recombinant antibodies to human PD-1 overexpressing 293F cells**

| Unique ID | Antibody concentration | Antibody Concentration |
|---|---|---|
| | 10µg/mL | 50µg/mL |
| PD1-A1 | 5.46% | 24.22% |
| PD1-A2 | 24.66% | 76.78% |
| PD1-A3 | 2.07% | 8.12% |
| PD1-C1 | 20.18% | 65.30% |
| PD1-C2 | 9.53% | 35.18% |
| PD1-C3 | 6.93% | 27.01% |
| PD1-C4 | 2.94% | 7.90% |
| PD1-C5 | 8.34% | 31.05% |
| PD1-C6 | 1.14% | 5.23% |
| PD-L1(His) | 20.52% | 67.90% |

### Example 9: PD-1/PD-L1 blocking assay

The PD-1/PD-L1 Blockade Bioassay was used to evaluate the blocking activity of nanobodies against the binding of PD-1 to PD-L1:
1)aAPC/CHO-K1 cells stably expressing human PD-L1 were seeded in 96-well plates at 5×10⁴ cells per well and cultured overnight.
2)Recombinant antibodies were added to 96-well plates together with Jurkat effector cells (1×10⁵ cells/well) stably expressing human PD-1 and NFAT-Luc. The cells were co-cultured with PD-L1-expressing aAPC/CHO-K1 cells for 6 hours.
3)Luciferase substrate was added, and the plate was incubated at room temperature for 10 min. Luminescence signals were measured with a plate reader, and data were analyzed using GraphPad Prism.

Firstly, a single-concentration blockade assay was performed at a antibody concentration of 10 µg/mL. The results (Table 6) indicated that compared with the positive control Keytruda (full-length IgG antibody), PD1-A2, PD1-C1 and PD1-C6 (VHH-His recombinant antibodies) could block the binding between PD-1 and PD-L1 and restore the TCR signaling pathway.

Further, concentration gradient assays were conducted for PD1-A2 and PD1-C1. The initial concentration was set at 30 µg/mL, followed by 3-fold serial dilution to generate a total of 9 concentration points. The dose-response blocking results (FIG. 2) showed that the PD1-C1 recombinant antibody exhibited complete blocking activity against PD-1/PD-L1 interaction, with an IC₅₀ of 0.68 µg/mL; the IC₅₀ of Keytruda for blocking PD-1/PD-L1 binding was 0.57 µg/mL.

**Table 6. PD-1/PD-L1 Blockade Activity of VHH-His Recombinant Antibodies (10 µg/mL)**

| Unique ID | Blocking percentage |
|---|---|
| PD1-A1 | 1.8% |
| PD1-A2 | 50.2% |
| PD1-A3 | 0.9% |
| PD1-C1 | 97.6% |
| PD1-C2 | 0% |
| PD1-C3 | 5.7% |
| PD1-C4 | 0% |
| PD1-C5 | 4.2% |
| PD1-C6 | 20.9% |
| Keytruda | 100% |

### Example 10: ELISA Binding Analysis of PD1-A2 and PD1-C1 with Cynomolgus Monkey and Mouse PD-1 Extracellular Domain Recombinant Proteins

The binding affinity of VHH-His recombinant antibodies PD1-A2 and PD1-C1 to cynomolgus monkey and mouse PD-1 extracellular domain recombinant proteins was determined by ELISA. Briefly, monkey or mouse PD-1 extracellular domain recombinant proteins (His tag) were coated onto 96-well ELISA plates at 100 ng/well and incubated overnight at 4 °C.

The VHH-His recombinant antibodies were serially diluted to gradient concentrations (0.01-10 µg/mL) and incubated with the coated antigens. HRP-conjugated anti-VHH secondary antibody was used for chromogenic reaction, and the absorbance at 450 nm was detected with a microplate reader.

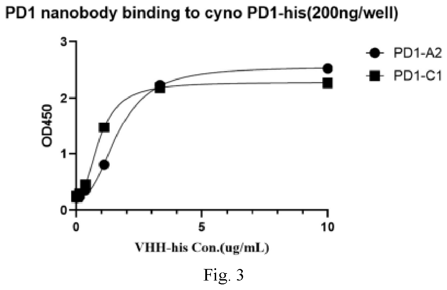

The experimental results (Table 7 and FIG. 3) indicated that the EC₅₀ values of PD1-A2 and PD1-C1 binding to cynomolgus monkey PD-1 extracellular domain recombinant protein were 1.69 µg/mL and 0.93 µg/mL, respectively. In contrast, neither PD1-A2 nor PD1-C1 exhibited specific binding to mouse PD-1 extracellular domain recombinant protein.

**Table 7. Binding affinity of VHH-His recombinant antibodies to cynomolgus monkey PD-1 (hFc tag)**

| Unique ID | Binding affinity to monkey PD-1 |
|---|---|
| PD1-A2 | 1.69 |
| PD1-C1 | 0.93 |

The above is only a preferred embodiment of the present invention. It should be pointed out that for ordinary technicians in this technical field, several improvements and modifications can be made without departing from the principle of the present invention. These improvements and modifications should also be regarded as the scope of protection of the present invention.

## Claims

1. An anti-PD-1 antibody (e.g., a nanobody), **characterized in that** the anti-PD-1 antibody comprises a CDR in any one of SEQ ID NOs: 64-72, exemplarily, the antibody comprises a complementary determining region CDR, and the complementary determining region CDR comprises any one of the following groups:
(1) CDR1 as set forth in SEQ ID NO: 1, CDR2 as set forth in SEQ ID NO: 2, and CDR3 as set forth in SEQ ID NO: 3;
(2) CDR1 as set forth in SEQ ID NO: 8, CDR2 as set forth in SEQ ID NO: 9, and CDR3 as set forth in SEQ ID NO: 10;
(3) CDR1 as set forth in SEQ ID NO: 15, CDR2 as set forth in SEQ ID NO: 16, and CDR3 as set forth in SEQ ID NO: 17;
(4) CDR1 as set forth in SEQ ID NO: 22, CDR2 as set forth in SEQ ID NO: 23, and CDR3 as set forth in SEQ ID NO: 24;
(5) CDR1 as set forth in SEQ ID NO: 29, CDR2 as set forth in SEQ ID NO: 30, and CDR3 as set forth in SEQ ID NO: 31;
(6) CDR1 as set forth in SEQ ID NO: 36, CDR2 as set forth in SEQ ID NO: 37, and CDR3 as set forth in SEQ ID NO: 38;
(7) CDR1 as set forth in SEQ ID NO: 43, CDR2 as set forth in SEQ ID NO: 44, and CDR3 as set forth in SEQ ID NO: 45;
(8) CDR1 as set forth in SEQ ID NO: 50, CDR2 as set forth in SEQ ID NO: 51, and CDR3 as set forth in SEQ ID NO: 52;
(9) CDR1 as set forth in SEQ ID NO: 57, CDR2 as set forth in SEQ ID NO: 58 and CDR3 as set forth in SEQ ID NO: 59.

2. The anti-PD-1 antibody according to claim 1, **characterized in that** the antibody further comprises a framework region FR, wherein the framework region FR comprises any one of the following groups:
(1) FR1 as set forth in SEQ ID NO: 4, FR2 as set forth in SEQ ID NO: 5, FR3 as set forth in SEQ ID NO: 6, and FR4 as set forth in SEQ ID NO: 7;
(2) FR1 as set forth in SEQ ID NO: 11, FR2 as set forth in SEQ ID NO: 12, FR3 as set forth in SEQ ID NO: 13, and FR4 as set forth in SEQ ID NO: 14;
(3) FR1 as set forth in SEQ ID NO: 18, FR2 as set forth in SEQ ID NO: 19, FR3 as set forth in SEQ ID NO: 20, and FR4 as set forth in SEQ ID NO: 21;
(4) FR1 as set forth in SEQ ID NO: 25, FR2 as set forth in SEQ ID NO: 26, FR3 as set forth in SEQ ID NO: 27, and FR4 as set forth in SEQ ID NO: 28;
(5) FR1 as set forth in SEQ ID NO:32, FR2 as set forth in SEQ ID NO:33, FR3 as set forth in SEQ ID NO:34, and FR4 as set forth in SEQ ID NO:35;
(6) FR1 as set forth in SEQ ID NO:39, FR2 as set forth in SEQ ID NO:40, FR3 as set forth in SEQ ID NO:41, and FR4 as set forth in SEQ ID NO:42;
(7) FR1 set forth in SEQ ID NO:46, FR2 set forth in SEQ ID NO:47, FR3 set forth in SEQ ID NO:48, and FR4 set forth in SEQ ID NO:49;
(8) FR1 set forth in SEQ ID NO:53, FR2 set forth in SEQ ID NO:54, FR3 set forth in SEQ ID NO:55, and FR4 set forth in SEQ ID NO:56;
(9) FR1 as set forth in SEQ ID NO:60, FR2 as set forth in SEQ ID NO:61, FR3 as set forth in SEQ ID NO:62, and FR4 as set forth in SEQ ID NO:63.

3. An anti-PD-1 antibody, **characterized in that** the anti-PD-1 antibody comprises any of the following amino acid sequences or its humanized sequence or a sequence having a sequence identity of more than 90%: SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71 or SEQ ID NO: 72.

4. A polynucleotide, **characterized in that** the polynucleotide is a polynucleotide encoding the anti-PD-1 antibody according to any one of claims 1 to 3.

5. A recombinant expression vector, **characterized in that** the recombinant expression vector comprises the polynucleotide according to claim 4.

6. A recombinant host cell, **characterized in that** the recombinant host cell contains the polynucleotide according to claim 4, or contains the expression vector according to claim 5.

7. The method for preparing the anti-PD-1 antibody according to any one of claims 1 to 3, **characterized in that** it comprises the following steps:
inserting a polynucleotide encoding the anti-PD-1 antibody according to any one of claims 1 to 3 into an expression vector to obtain a recombinant expression vector;
introducing the recombinant expression vector into a host cell to obtain a recombinant host cell;
cultivating the recombinant host cell to obtain a culture;
the culture was purified to obtain anti-PD-1 antibody.

8. A bispecific antibody, **characterized in that** the bispecific antibody comprises the anti-PD-1 antibody according to any one of claims 1 to 3 and a second antibody.

9. Use of the anti-PD-1 antibody according to any one of claims 1 to 3 or the bispecific antibody according to claim 8 in the preparation of a drug for preventing and/or treating cancer, or in detecting PD-1 protein.

10. A pharmaceutical composition, **characterized in that** the pharmaceutical composition comprises: the anti-PD-1 antibody according to any one of claims 1 to 3 or the bispecific antibody according to claim 8; and a pharmaceutically acceptable excipient.

11. A kit for detecting PD-1 protein, **characterized in that** the kit comprises: the anti-PD-1 antibody according to any one of claims 1 to 3 or the bispecific antibody according to claim 8; and a detection-acceptable reagent.

12. A chimeric antigen receptor (CAR), the extracellular domain of which comprises the anti-PD-1 antibody according to any one of claims 1 to 3.

13. A cell comprising or expressing the CAR of claim 12, such as a T cell or a NK cell.

14. A method for treating cancer, comprising administering to a subject the antibody of any one of claims 1 to 3 or a cell comprising or expressing a CAR thereof, such as a T cell or a NK cell.
